# EUROPEAN PATENT APPLICATION

(11) **EP 1 087 018 A1**
(43) Date of publication of application: **28.03.2001**
(21) Application number: 00117771.6
(22) Date of filing: 18.08.2000
(51) Int. Cl.: C12P 7/02, C12P 7/40, C12P 7/62, C12P 13/02, C12P 41/00

(54) **Aminoacylases for acylation of alcohols and amines**

(30) Priority: 24.09.1999 EP 99118844
(71) Applicant: Degussa-Hüls Aktiengesellschaft, 60287 Frankfurt am Main (DE)
(72) Inventor: Bakker, Martin, 1051 TW Amsterdam (NL); van Rantwijk, Frederik, 2742 DL Waddinxveen (NL); Sheldon, Roger Arthur, 2281 VA Rijswijk (NL)

(57) **Abstract**

A process for the acylation of racemic alcohols or amines or their cleavage by the aid of aminoacylases is described.

Amines and alcohols can be acylated by aminoacylases. So far only amino acids and alcohols have proved to be suitable substrates. But alcohols are poorly transformed.

Immobilised aminoacylases transform alcohols enantioselectively into esters to an extent that the reaction can be adopted on technical scale in an economically manner. Aminoacylases turnover amines accordingly.

## Description

The present invention is directed to a process for the acylation of racemic alcohols or amines or their cleavage by the aid of aminoacylases.

Enantioselective synthesis is a versatile tool to produce chiral non-racemic organic compounds in organic chemistry. Its applicability in the production of substances on technical scale becomes more and more important due to the ever growing need to provide purer and cheaper products. Thus, there is still a demand for new and superior processes in chemical industries.

One straightforward method of industrial enantioselective synthesis is the transformation of compounds by enzymes. Aminoacylases are well known in the art to be suitable catalysts and are incorporated in many industrial applications (e.g. DE 19546532; Bommarius et al. Ann. N. Y. Acad. Sci. 1992, 672, 126). On the other hand aminoacylases are deemed to be ineffective in transforming amines to amides enantioselectively and vice versa, respectively.

Enzymes to be used advantageously with respect to alcohols are lipases in this regard (Lalonde et al. Methods in Enzymology 1997, 286, 443-464). Herradon et al. published the possibility to transesterify enantioselectively ±1-phenylethanol with vinylbutyrate by an acylase of Aspergillus melleus (Synlett 1997, 367-370).

Above mentioned processes with respect to aminoacylases have the disadvantage to provide undesirably low productivity for the transformation of alcohols to be implemented on industrial scale or are not known in the art with regard to transformations of amines so far.

Therefore, it was an object of this invention to provide a catalytic process for the enantioselective acylation of racemic alcohols or the saponification of racemic esters and the enantioselective acylation of racemic amines or the cleavage of racemic amides in a manner to adopt this process on industrial scale, which requires a high efficiency regarding yields, purities and costs of products.

This task is realised by a process characterised by claim 1. Preferred embodiments of this invention are subject to claims depending from claim 1.

The catalytic enantioselective acylation of racemic alcohols or enantioselective saponification of racemic esters can be done advantageously by reaction with immobilised aminoacylases. Surprisingly, free or imobilised aminoacylases can be taken for enantioselective acylation of racemic amines or enantioselective cleavage of racemic amides, too. So far the theory revealed that only amino acids are deemed to be proper substrates for aminoacylase transformation (A. S. Bommarius et al., Tetrahedron; Asymmetry, 1997, Vol. 8, 3197-3200).

It is a unique feature of aminoacylases to be able to transform alcohols and amines according to the invention. Preferably, aminoacylases from Aspergillus are used. More advantageously, the process can be performed with aminoacylases taken from Aspergillus melleus or Aspergillus oryzae.

The mode of immobilisation of instant enzymes can be chosen according to the knowledge of the skilled worker (Bhavender P. Sharma, Lorraine F. Bailey and Ralph A. Messing, "Immobilisierte Biomaterialiern - Techniken und Anwendungen", Angew. Chem. 1982, 94, 836-852). Preferably, the enzymes are immobilised by lyophilisation (Dordick et al. J. Am. Chem. Soc. 194, 116, 5009-5010; Okahata et al. Tetrahedron Lett. 1997, 38, 1971-1974; Adlercreutz et al. Biocatalysis 1992, 6, 291-305) . Most preferred is a lyophilisation using surfactants like aerosol OT or polyvinylpyrrolidone or polyethylenglycol (PEG) or Brij 52 (Diethylenglycol-monocetylether) (Goto et al. Biotechnol. Techniques 1997, 11, 375-378.

To cleave amides or to saponify esters water has to be present in the reaction mixture or the reaction can be totally run in water and the pH-value has to be kept constant in a range from 5 to 9, preferably around 7(±1). For the reversed reactions an organic solvent is preferably used. In case of acylation the process can advantageously be conducted in a non-polar organic solvent. More preferably an aliphatic or aromatic hydrocarbon, like hexane, toluene can be used as solvent. Most preferably and totally surprisingly the acylation can be conducted without additional solvents.

For substrates all amines or amides or alcohols or esters can be used as long as they possess at least one centre of chirality. The enantioselectivity will be larger the narrower this centre is connected to the centre of reaction. Advantageously, the centre of chirality is related to the centre of reaction. Therefore, a secondary alcohol or amine with adjacent secondary centre can be acylated preferably.

Within the preferred amines and alcohols those are most preferred, which are connected to aromatic residues. Thus an aromatic alcohol or amine with adjacent secondary centres can be used most advantageously.

The instant invention can be performed in an enzyme-membrane reactor like mentioned in Wandrey et al. in Jahrbuch 1998, Verfahrenstechnik und Chemieingenieurwesen, VDI S. 151ff.; Wandrey et al. in Applied Homogeneous Catalysis with Organometallic Compounds, Vol. 2, VCH 1996, S.832 ff.; Kragl et al., Angew. Chem. 1996, 6, 684f or DE 197 27 715.

In the following table 1 results are shown of reactions of rac-1-phenylethanol with activated esters in the presence of aminoacylase of Aspergillus melleus.

| Activated ester | Rate^{a} | Conversion (%) | Time (d) | ee (%) |
|---|---|---|---|---|
| vinylacetat | 52,6 | 50 | 3 | >99 |
| vinylpropionate | 67,0 | 50 | 2 | >99 |
| vinylbutyrate | 109,7 | 50 | 1,25 | >99 |
| vinylacrylate | 26,6 | 37 | 7 | >99 |
| vinylcrotonate | 40,2 | 45 | 7 | >99 |
| vinylhexanoate | 15,3 | 50 | 4 | >99 |
| vinyloctanoate | 14,6 | 50 | 4 | >99 |
| vinyldodecanoate | 13,7 | 50 | 4 | >99 |
| vinylbenzoate | 1,9 | 13,5 | 7 | 72 |
| vinyllaurate | 35 | 50 | 6 | >99 |
| isoprenylacetat | 1,2 | 8 | 7 | 48 |
| methylbutyrate | 4,3 | 11,6 | 7 | >99 |

| | | | | |
|---|---|---|---|---|
| ^{a}: reaction rate in µmol product*(min*mg protein)⁻¹ | | | | |

The following table 2 shows the results of transformations of different aromatic alcohols with vinylbutyrate and aminoaclase from Aspergillus melleus in hexane

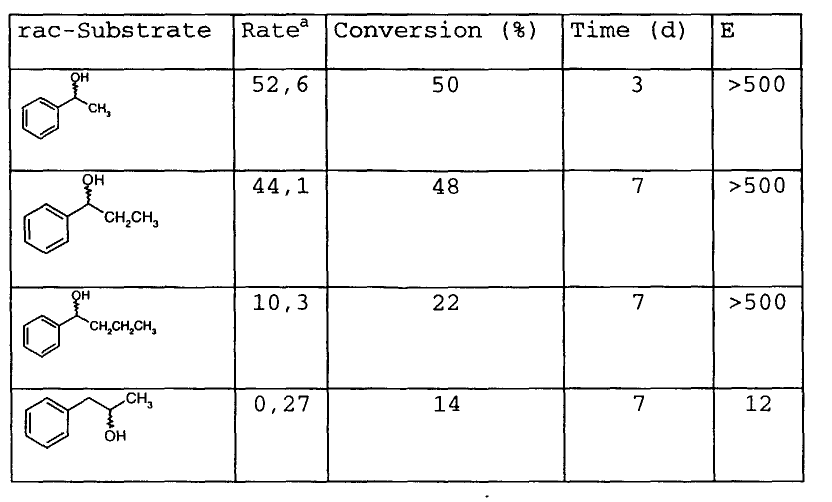

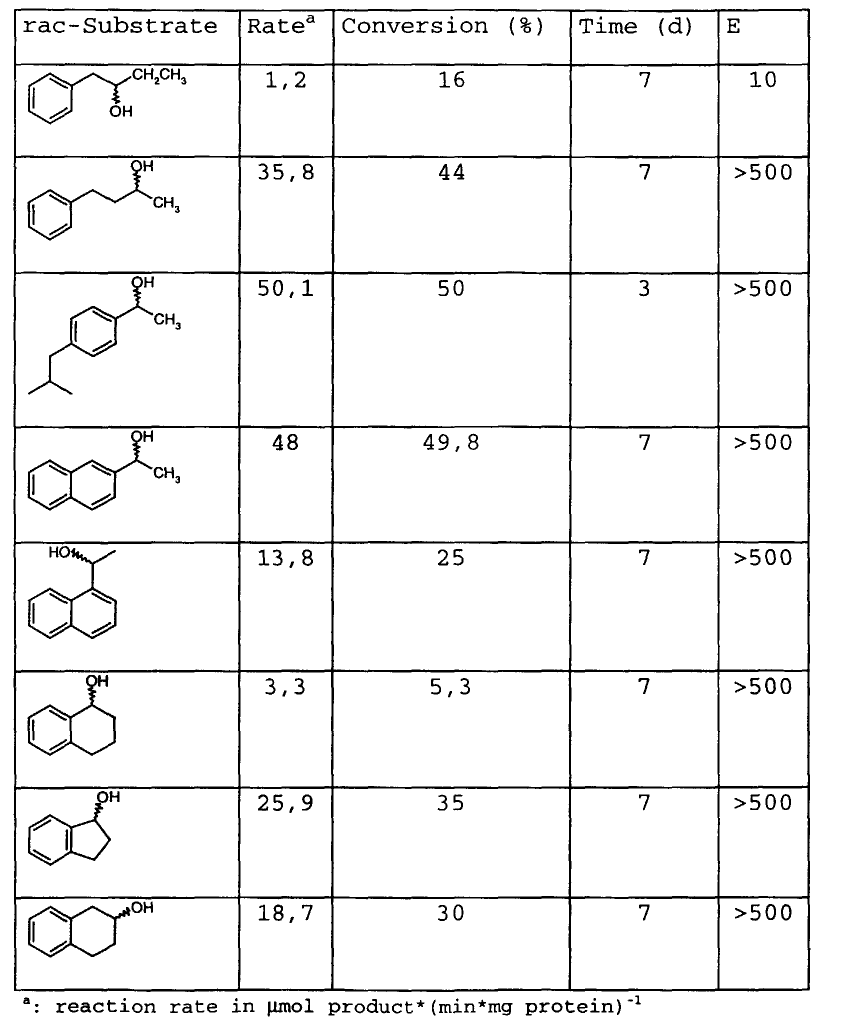

The following table 3 reveals the increase in productivity of surfactant coated enzymes and native enzymes

| Aminoacylase | Hydrolytic activity (Umg⁻¹)^{a)} | Transesterification (µmolmin⁻¹mg⁻¹) | STY (gl⁻¹d⁻¹) | Productivity (gg⁻¹h⁻¹) |
|---|---|---|---|---|
| | | | | |

| A. oryzae | | | | |
|---|---|---|---|---|
| Native | 11,2 | 0,2 | 45 | 0,6 |
| PVP-complex | 3,89 | 2,4 | 70 | 6,4 |
| AOT-complex | 4,38 | 74 | 2104 | 127,2 |

| A. melleus | | | | |
|---|---|---|---|---|
| Native | 100 | 0,4 | 47 | 1,2 |
| PVP-complex | 6,22 | 3,6 | 50 | 8,8 |
| AOT-complex | 5,33 | 20,0 | 376 | 41,1 |

| | | | | |
|---|---|---|---|---|
| ^{a)}; one unit will hydrolyse 1 µmol N-acetyl-L-methionine per minute | | | | |

It can be deduced from above mentioned results that aminoacylases are deemed to be excellent catalysts for the enantioselective transesterification of racemic alcohols to their corresponding esters in their immobilised form. Due to reversibility of instant reactions the saponification is also possible purely by rendering the thermodynamic reaction conditions. Amines are transformed accordingly into their enantiomerically enriched amides and vice versa.

Enantioselective means within the framework of this invention that one antipode is the major component in the mixture of both antipodes.

Immobilisation means according to this invention the crosslinking of enzymes (clecs); the adsorption of enzymes on special devices (e.g. nitrocellulose) or other techniques known to the artisan to harbour enzymes on polymerically enlarged substrates, like e.g. surfactant coating techniques (Goto et al. Biotechnol. Prog. 1994, 10, 263-268; Kamiya et al. Biotechnol. Prog. 1995, 11, 270-275; Okahata et al. Tibtech February 1997, 15, 50-54; Fishman et al. Biotechnol. Lett. 1998, 20, 535-538).

Aminoacylases from A. melleus and A. oryzae are commercially available.

Amines according to the invention mean primary amines, which do not possess an α-carboxylic group.

### Examples:

### Analysis and Equipment:

The lyophilisation of enzyme-surfactant solutions was performed in rubber sealed freeze-dry flasks (Salm en Kipp bv, 300 ml). The reactions were monitored by reversed phase HPLC analysis. Polyvinylpyrrolidone K30 (PVP), Aerosol OT (10% v/v) (AOT) were obtained from Aldrich.

### Enzyme lyophilisation:

To prepare surfactant coated protein 5 g of dry weight PVP K30 was dissolved in 50 ml buffer (100mM KH₂PO₄, pH 8.0) and mixed with 1 g catalyst. After achieving homogeneous enzyme solution, the liquid was frozen at -40 °C and the resulting frozen aqueous layer was removed under vacuum during 16 h. The activity of aminoacylases preparations was measured using the standard hydrolysis of N-acetyl-L-methionine as the assay.
In the case of AOT, 28 g of emulsion (10% Aerosol) was mixed with 50 ml buffer (100 mM KH₂PO₄), pH 8.0) and when 1 g of enzyme was dissolved the liquid was frozen and dried under vacuum.

### Transesterification reactions:

100 mg of rac-1-phenylethanol (0.8 mmol) were combined with 1.5 eq. activated ester, 100 mg catalyst and 5 ml hexane at room temperature (see table 1/2). During the first 5 h the initial rate was measured by following the course of the reaction with chiral HPLC. For the surfactant-coated catalyst (see table 3) the reactions were performed without additional solvents, solubilising 200 mg of surfactant-prepared catalyst in 3 g of rac 1-phenylethanol (24.6 mmol) and 1.1 eq. vinylbutyrate. The samples for analysis were centrifuged to remove the surfactant/protein layer from substrate/product. The excess of vinylbutyrate was evaporated and the residue was purified by silica gel column chromatography to afford (S)-alcohol and (R)-ester.

## Claims

1. Process for the enantioselective acylation of racemic alcohols or enantioselective saponification of racemic esters with immobilised aminoacylases or enantioselective acylation of racemic amines or enantioselective cleavage of racemic amides with free or immobilised aminoacylases.

2. Process according to claim 1,
characterised in that
aminoacylases of Aspergillus melleus or Aspergillus oryzae are used.

3. Process according to claim 1 and/or 2,
characterised in that
the catalysts are immobilised by lyophilisation with surfactants like aerosol OT or polyvinylpyrrolidone.

4. Process according to one or more of claims 1 to 3,
characterised in that
in case of acylation the process is conducted in a non-polar organic solvent.

5. Process according to claim 4,
characterised in that
an aliphatic or aromatic hydrocarbon is used as solvent.

6. Process according to one or more of claims 1 to 3,
characterised in that
in case of the acylation the process is conducted without additional solvents.

7. Process according to one or more of claims 1 to 6,
characterised in that
a secondary alcohol or amine with an adjacent secondary centre is acylated.

8. Process according to claim 7,
characterised in that
an aromatic alcohol or amine is used.

9. Process according to one or more of the preceding claims, characterised in that
the reaction is performed in an enzyme-membrane reactor.
